# EUROPEAN PATENT SPECIFICATION

(11) **EP 2 960 223 B1**
(45) Date of publication and mention of the grant of the patent: **18.12.2019**
(21) Application number: 14173883.1
(22) Date of filing: 25.06.2014
(51) Int. Cl.: C07C 5/333, C07C 5/52

(54) **An endothermic gas phase catalytic dehydrogenation process**
Katalytisches Dehydrierungsverfahren mit endothermer Gasphase
Processus de déshydrogénation catalytique de phase de gaz endothermique

(43) Date of publication of application: 30.12.2015
(73) Proprietor: Borealis AG, 1220 Wien (AT)
(72) Inventor: Mathivanan, Guhan, 4020 Linz (AT); Rumetshofer, Thomas, 4351 Saxen (AT); Dicke, René, 4060 Leonding (AT); Leitner, Andreas, 4240 Freistadt (AT)
(74) Representative: Maikowski & Ninnemann Patentanwälte Partnerschaft mbB

(56) References cited:
- EP-A1- 0 221 332
- WO-A1-92/19575
- WO-A1-95/30635
- US-A- 942 944
- US-A- 3 267 170
- US-A- 4 546 204

## Description

The present invention relates to an endothermic catalytic dehydrogenation process according to the preamble of claim 1.

### Description

The invention is concerned with an endothermic hydrocarbon process, particularly for catalytic dehydrogenation of paraffinic and other hydrocarbons such as propane dehydrogenation (reaction 1) or butane dehydrogenation (reaction 2) or i-butane dehydrogenation (reaction 3):

*C*₃*H*₈ ⇔ *C*₃*H*₆ + *H*₂ (1)

*C*₄*H*₁₀ ⇔ *C*₄*H*₆ + 2*H*₂ (2)

*i* - *C*₄*H*₁₀ ⇔ *i* - *C*₄*H*₈ + *H*₂ (3)

Dehydrogenation of hydrocarbons, in particular aliphatic hydrocarbons, to convert them into to their respective olefins is a well-known process. For example, the hydrocarbons propane, butane, isobutane, butenes and ethyl benzene are well known catalytically dehydrogenated to produce the respective propylene, butene, isobutene, butadiene and styrene. Dehydrogenation reactions are strongly endothermic and thus, an increase of the heat supply favours the olefin conversion.

One well known dehydrogenation process is the Houdry CATOFIN® process in which an aliphatic hydrocarbon is passed through a dehydrogenation catalyst bed where the hydrocarbon is dehydrogenated to the respective olefin, the olefin is flushed from the bed, the catalyst is regenerated and reduced, and the cycle is repeated (US 2,419,997).

Some other well-known dehydrogenation technologies are Oleflex, Uhde-STAR and BASF-Linde process. Oleflex and CATOFIN technologies are adiabatic processes where the catalyst bed is heated directly. Uhde-STAR and BASF-Linde technologies are isothermal processes where the catalyst bed is heated indirectly.

CATOFIN propane dehydrogenation process is a cyclic process where during regeneration and reduction steps, heat is supplied to the catalyst bed and during dehydrogenation step catalyst bed cools down due to the endothermic dehydrogenation reaction. The upper section of the catalyst gets most of the heat during regeneration and reduction steps and supplies most of the heat to the reaction during the dehydrogenation reaction. On the other hand the heat consumed and supplied by the lower sections of the bed is quite low compared to the upper sections. Propylene production is normally controlled by equilibrium at the bottom section.

Another well-known process is CATADIENE® process in which butanes and butenes are dehydrogenated to produce butadiene.

Typically the catalyst is heated by contact with a heated gas, usually air. The aliphatic hydrocarbon such as propane is passed through the hot catalyst bed which supplies the heat for the dehydrogenation reaction. Since the dehydrogenation reaction is endothermic and the process is adiabatic, the temperature of the catalyst gradually drops during the dehydrogenation cycle and causes a decrease in hydrocarbon conversion rate. Particularly the temperature at the top of the catalyst bed decreases by as much as 100°C (US 2007/0054801 A1).

In order to reheat the catalyst bed and remove coke that has deposited on the catalyst during the dehydrogenation step, the reactor is purged of hydrocarbon and undergoes a regeneration step with air heated to temperatures up to 700°C. Heat is provided to the bed by the hot air that passes through the bed and also by the combustion of coke deposits on the catalyst. After reheating and regenerating the catalyst and before putting the reactor back on stream, the oxidized catalyst must be reduced by passing a reduction gas such as hydrogen or hydrocarbon through the catalyst bed. This also supplies additional heat by the oxidation of the reducing gas (US 2007/0054801 A1).

The question of additional heat supply to the reactor bed in order to compensate for the heat consumption during the strong endothermic reaction has been addressed in the past and different approaches have been suggested.

For instances US 6,392,113 B1 proposes to react the hydrocarbon feed partially in a catalytic pre-reactor and reheating the exiting hydrocarbon stream from pre-reactor to the same preheat temperature prior to introduction into the main catalytic reactor. Reheating can be done either by exiting regeneration air or by a separately fired heater.

US2006/0004241 A1 discloses a way to decrease temperature gradient in isothermal propane dehydrogenation process. This patent proposes to dilute the catalyst bed with inert particles at the beginning of the catalyst bed to decrease the temperature gradient in the section of catalyst bed for the isothermal propane dehydrogenation process.

WO2007/018982 A1 uses two different catalyst zones for adiabatic, non-oxidative hydrocarbon dehydrogenation process. The first zone of catalyst has higher activity and higher capacity to produce more coke than the second layer. The combustion of coke in turn provides extra heat.

A similar approach is applied by US 4,560,824 A, which proposes diluting an isobutane feed stream with n-butane and recycling unreacted products and n-butene from the dehydrogenation zone. This increases the coke production.

WO 95/30635 A1 describes a process for producing olefins by trans-hydrogenation. In this process, a saturated hydrocarbon is co-fed with an olefin whereby coupling the endothermic reaction of dehydrogenation with a hydrogenation of olefins which is an exothermic reaction.

US 4,546,204 A discloses a process for the production of MTB ether wherein the process comprises contacting a mixture comprising an ethylene enriched stream and an iso-butane enriched stream with a transhydrogenation catalyst to give a hydrocarbon mixture comprising iso-butene and ethane.

EP 221332 A1 discloses a trans-hydrogenation process whereby a mixture of at least two hydrocarbons, at least one of which is an alkane and at least one of which is an alkene, is fed to a catalyst bed and in the subsequent reaction the alkane converts to an alkene and the alkene converts to an alkane. The efficiency of such a trans-hydrogenation process is improved applying certain reaction conditions such as a specific pressure, a temperature of the catalyst between 400 to 600° Celsius, a certain volume of hydrogen acceptor. The hydrogen acceptor may be any alkene, in particular ethylene and propylene.

US 3,267,170 A relates also to a process for forming olefins by hydrogen transfer wherein a hydrocarbon containing more than 3 carbon atoms is reacted with a hydrogen accepting olefin in the presence of the supported catalyst comprising a sulfide of a metal such as a molybdenum sulfide. For instance, according to example 1, a mixture of ethylene and propane are fed together into the reaction zone at a certain feed rate. When doing so, an improved conversion to olefins is observed.

As can be seen from the prior art there are several possibilities to increase the heat during the dehydrogenation process, in particular by increasing the hydrocarbon feed temperature, increasing air inlet temperature and increasing air flow rate. Additional heat can also be provided by injecting and combusting a fuel gas with regeneration air and increasing flow rate of said injection gas.

However, the above described approaches for increasing the heat supply in the catalytic bed have several drawbacks. An increase of temperature of the hydrocarbon feed could create cracking of hydrocarbons to lighter products and coking in the pipelines. An increase of the air inlet temperature or injection gas flow rate in order to increase the temperature in the catalyst layer could accelerate catalyst deactivation.

Furthermore, once designed, a plant runs to the limit of air handling equipment. Increasing air flow requires major capital expenditures (US 6,392,113 B1).

Also significant temperature differences can be observed between the top of the catalyst zone and bottom of the catalyst zone due to heat exchange of the alkane feed with the catalyst and endothermic dehydrogenation reaction. Due to this temperature drop the catalyst at the top to deactivate faster than the catalyst at the bottom.

In order to tackle that problem WO2007/030298 A1 suggests an improved catalyst bed system. In one part of the catalyst bed, the propane dehydrogenation catalyst is mixed with any inert material which does not produce heat during the whole process and in another part of the bed, propane dehydrogenation catalyst along with normal inert material is mixed with a new material which is catalytically inert with respect to dehydrogenation, cracking, coking reactions, but generates heat when it is exposed to reducing and/or oxidizing reaction conditions. Though it gives advantage in providing isothermality to a catalyst bed, it also provides inconvenience in the case of if one wants to change or adapt the isothermality, since it cannot be changed while the process is operating.

Thus, it would be of an advantageous to provide a process for improving conversion of a gas phase dehydrogenation process without having the above described drawbacks. It would be in particular of an advantageous to reduce the temperature drop within the reactor, in particular in the catalyst zone of the dehydrogenation reactor.

This objective is being solved by a process according to claim 1.

Accordingly, an endothermic catalytic propane dehydrogenation process is provided which is conducted in gas phase in at least one reactor system comprising at least two reactors that are connected in series and comprise at least one catalyst bed comprising at least one inorganic catalytic material and at least one first inert material.

The dehydrogenation process comprises the steps of:
- feeding at least one first stream comprising propane (as alkane of the general formulae I CₙH₂ₙ₊₁R¹ with n =3 and R¹ = H) into the first reactor as front feed, and
- feeding at least one second stream comprising ethylene (as olefin of the general formulae II CₘH₂ₘ with ₘ = 2) into the gas stream leaving the first reactor at at least one location between the first reactor and the second reactor;
wherein the gas stream leaving the first reactor and the at least one second stream comprising ethylene are mixed in / or before entering at least one heater arranged between first reactor and second reactor and heated in the heater before entering the second reactor.

Thus, propane as alkane in the at least one first stream S1 imperatively comprises at least one more carbon atom than ethylene as olefin in the at least one second stream S2.

Thus, in the present process the alkane or hydrocarbon to be dehydrogenated can be co-fed with an olefin, which has at least one carbon atom less than the alkane to be dehydrogenated. For example, propane (C3) to be dehydrogenated to propene is co-fed with ethene (C2).

By co-feeding an olefin with the alkane feed or supplying an olefin to a section of the reactor can increase the conversion of the dehydrogenation reaction for several reasons. Firstly, the olefin supplied reacts with the hydrogen, which is formed in dehydrogenation reactions of the alkane, in an exothermic hydrogenation reaction. Co-feeding olefin provides additional heat to the catalyst bed and due to that the temperature drop normally observed in dehydrogenation catalyst bed is reduced.

Co-feeding olefin has additional flexibilities such as either it can be fed at the top of catalyst zone after certain dehydrogenation time to decrease the temperature drop at the top or it can be fed directly to the lower sections of the bed to achieve a more isothermal bed.

In a further variant the dehydrogenation process comprises the steps of:
- feeding at least one first stream S1 comprising propane as alkane of the general formulae I CₙH₂ₙ₊₁R¹ with n =3 and R¹ = H into the at least one reactor for a total alkane feeding time tS₁,
- feeding at least one second stream S2 comprising ethylene as olefin of the general formulae II CₘH₂ₘ with ₘ =2 into the at least one reactor for a total feeding time tS₂,
- wherein the ratio z of total olefin feeding time tS₂ and total alkane feeding time tS₁ is between 0.001 and 0.95, preferably 0.01 and 0.5, most preferably between 0.05 and 0.1.

Within the context of the present invention it will be understood that the ratio of total olefin feeding time tS₂ and total feeding time tS₁ is imperatively below 1. This means that the total olefin feeding time tS₂ is always smaller or shorter than the total alkane feeding time tS₁. Thus, the beginning of olefin feeding is imperatively delayed in respect to alkane feeding time. The olefin is not immediately fed together with the alkane stream to the dehydrogenation reactor, but rather with a time delay. The olefin stream S2 is only fed to the reactor after dehydrogenation reaction of the alkane in the catalyst bed has already started.

This can also be described such that the alkane feeding is started at a time point tS₁₍₀₎ and ends at a time point tS_{1(E)} providing tS₁ = tS_{1(E)} - tS_{1(0);} and the olefin feeding is started at a time point tS₂₍₀₎ and ends at a time point tS_{2(E)} providing tS₂ = tS_{2(E)} - tS₂₍₀₎.

The time point tS₂₍₀₎ for the start of feeding the olefin stream S2 is provided after time point tS₁₍₀₎ for the start of feeding the alkane stream S1 such that tS₂₍₀₎ = tS₁₍₀₎ + x, wherein x is any possible time interval above 0 sec. The time difference between feeding the alkane stream and the olefin stream can be of any x value, such for instance at least 30 sec, at least 1 min or at least 5 min (provided that the above ratio of tS₂ : tS₁ is full filled). For example, if x is 1 min and tS₁ is 10 min and tS₂ is 9 min then the ratio of tS₂ : tS₁ would be 0.9.

In an embodiment total continuous alkane feeding S1 (or dehydrogenation) begins at tS₁₍₀₎ and ends at tS_{1(E)}. Olefin feeding S2 is started at tS₂₍₀₎ after a certain dehydrogenation time tS₁₍₁), i.e. tS₁₍₁₎ = tS₂₍₀₎ = tS₁₍₀₎ + x = S_{1(E)} - tS_{2(E)}. The olefin feeding S2 may then be continued until the dehydrogenation reaction is ended, e.g. for instance until tS_{1(E)} is reached. In this case alkane and olefin are fed together starting at tS₂₍₀₎.

It is also possible to interrupt the olefin feeding S2 at any time value y, for instance at tS₂₍₁₎ = tS₂₍₀₎ + y for a time (or break) value z and to restart the olefin feeding at tS₂₍₂₎ = tS₂₍₀₎ + (y+z). Such an interruption of olefin feeding would have the advantage of reducing the overall coke formation.

It is furthermore possible to stop the olefin feeding before the end of dehydrogenation at tS_{1(E)}. For instance in the last minute of the dehydrogenation reaction there is no olefin feed necessary, since the additional heat created by the olefin hydrogenation would be lost.

In yet a further variant the dehydrogenation process comprises the steps of
- feeding at least one first stream S1 comprising propane as alkane of the general formulae I CₙH₂ₙ₊₁R¹ with n =3 and R¹ = H into the at least one reactor, and
- feeding at least one second stream S2 comprising ethylene as olefin of the general formulae II CₘH₂ₘ with ₘ =2 into the at least one reactor,
wherein the first alkane stream S1 is fed to the at least one reactor of the reactor system as front feed and the second olefin stream S2 is fed to the at least one reactor of the reactor system at at least one location alongside of the at least one reactor.

In a preferred embodiment the two process variants can be also combined, i.e. the time and local adaption of alkane and olefin feeding can be combined.

The two process variants of the present invention provide a desired isothermality of the overall dehydrogenation process. At present two types of isothermality are realized: a) either by decreasing the temperature drop that normally happens during the dehydrogenation step at a fixed catalyst position by feeding alkane and olefin at different time intervals or b) by decreasing the temperature gradient that normally exists along the length of the reactor by feeding alkane and olefin at different locations of the reactor.

Thus, in a single reactor system, olefin feeding can be done at the top of catalyst bed or at the deeper bed and either can be fed from the beginning of dehydrogenation step or after few minutes of the dehydrogenation step. The timing and the place to feed olefin can be chosen to achieve the desired isothermality.

In an embodiment of the processes the second olefin stream S2 is fed along the at least one reactor of the reactor system at at least one of the following locations: top of the catalyst bed, first half of the catalyst bed and second half of the catalyst bed.

A yet another effect of feeding an olefin stream (for example together with or delayed to the alkane feed or at any location alongside of the reactor) is an extension of the total dehydrogenation time tS₁. This is due to the fact that the olefin feeding reduces the temperature drop over the catalyst bed as described above.

In a further variant of the present process a layer of a second inert material, which may be the same as the first inert material or different from the first inert material, is arranged upstream and/or downstream of the catalyst bed.

One preferred arrangement is that the layer of a second inert material is disposed on top or on the upper surface of the catalyst bed which is usually arranged in a horizontal manner. The layer of second inert material and the catalytic material are in direct contact with each other. Tthe layer of the second inert material may have approximately a thickness D in a range between 10 cm and 100 cm, preferably 15 cm and 60 cm, most preferably between 20 and 40 cm.

Another possible arrangement is that the second inert material is arranged in an extra vessel which is being upstream of the dehydrogenation reactor. Thus, the layer of second inert material and the catalytic material are not in direct contact with each other; they are rather spatially separated. Thus, in another embodiment of the present catalyst bed system the predetermined volume of the second inert material is arranged in at least one extra vessel, which is arranged upstream of the reactor. In this case the volume of the second inert material in the at least one vessel may be between 15 and 180 tons, preferably 20 and 110 tons and most preferably 30 and 70 tons.

In an embodiment of the processes the second olefin stream S2 is fed to the at least one layer of a second inert material arranged upstream and/or downstream of the at least one catalyst bed in the at least one reactor.

In a variant of the process the temperature of the alkane feed and of the olefin feed are between 400 and 650 °C, preferably 500 and 650 °C, most preferably 550 and 650 °C, respectively.

The reaction temperatures in the catalyst bed may be between 500 and 1000°C, preferably between 500 and 800 °C, most preferably between 500 and 700 °C. The catalyst bed is heated by introducing a heat stream for heating and/or regenerating the catalytic material.

The heat stream preferably comprises a hot air stream or air feed and an injection gas feed. Thus, the temperature of the heat stream is preferably the temperature resulting from the combustion of air and injection gas. It is however in general conceivable to increase the heat input also by other measures. For instance, heat can be provided in a direct manner such as by combustion of fuel gas or in an indirect manner by heating air without combustion gas. It is furthermore conceivable to increase the heat input into the catalytic bed also by measures such as heating the reactor mantle. That means heating measures from the inside or the outside of the reactor are possible. It is also conceivable to add the heat within the reactor or before air enters the reactor.

The hot air stream may be fed at a rate between 100 and 500 Mt/hr, preferably between 150 and 400 Mt/hr, most preferably between 200 and 300 Mt/hr, whereby 210 Mt/hr is the typical applied feed rate.

The injection gas stream can be fed with a rate between 0.1 and 0.6 kg/ sec preferably between 0.1 and 0.4 kg/sec, most preferably between 0.1 and 0.2 kg/sec, whereby 0.125 kg/sec is the typical fed rate. Thereby the fed rate of the injection gas stream depends strongly on the operational mode as described above.

In general the present process may be conducted at a pressure in a range between 0,013 MPa (100 mmHg) to 0,1 MPa (750 mmHg).

The first alkane stream S1 may be fed at a rate between 20 and 60 Mt/hr, preferably between 25 and 50 Mt/hr, most preferably between 35 and 50 Mt/hr.

The flow rate of the olefin feed S2 is between 500 kg/h and 30 t/h, preferably between 1 and 20 t/h, most preferably between 2 and 10 t/h.

The ratio of the first alkane stream S1 and the second olefin stream S2 is between 50 and 1, preferably between 45 and 3, most preferably between 30 and 15. Thus, the first alkane stream S2 is preferably fed in excess to the olefin.

The inorganic catalytic material of the catalyst bed is preferably selected from a group consisting of chromium oxide, platinum, iron, vanadium and their respective oxides or a mixture thereof.

The first inert material of the catalyst bed is preferably selected from the group consisting of magnesium oxide, aluminium oxide, aluminium nitride, titanium oxide, zirconium dioxide, niobium oxide, aluminium silicate and others. The inert material may not only serve as heat storing material but also may have the function of a support system.

In the context of the present invention "inert material" is defined as a material which does not exhibit any catalytic effect in the dehydrogenation reaction, but may participate in other reactions such as cracking or coking which take place during dehydrogenation.

The catalyst bed comprises preferably 50 Vol% of a catalytic material and 50 Vol% of a first inert material. However, in case of isobutane dehydrogenation 70 Vol% catalytic materials is mixed with 30 Vol% inert materials (see US 2007/054801 A1).

A typical Chromium oxide dehydrogenation catalyst manufactured on an alumina support comprises from about 17wt% to about 22 wt% Cr₂O₃. These type of dehydrogenation catalyst are known for instance under the name Catofin® Standard catalyst (US 2008/0097134 A1). It is to be understood that the concept of the present process - namely the use of an extra inert layer - is applicable to any type of dehydrogenation catalyst and not only to the ones explicitly mentioned within the context of this application. Thus, all other commonly used dehydrogenation catalysts may also be applicable.

The catalyst bed is prepared by mixing or combining the catalytic material and the first inert material. The required amount of catalytic material is determined and is then mixed with a defined amount of first inert material. The catalyst bed is evacuated and reduced with hydrogen. Then an aliphatic hydrogen carbon such as propane, butane, isobutane or an aromatic alkane such as ethyl benzene is fed to the catalyst bed and is dehydrogenated upon contact with the catalytic material to the corresponding unsaturated alkanes such as propylene, butadiene, isobutene or styrene.

The present dehydrogenation process may be conducted in a single reactor with multiple tubes or in multiple parallel reactors as in CATOFIN process. Also it can be conducted in multiple serial reactors as in Oleflex process.

According to the invention the present dehydrogenation process is conducted in a reactor system comprising at least two reactors, which are connected in series, and which comprise at least one first catalyst bed comprising at least one inorganic catalytic material and at least one first inert material, respectively.

This arrangement can be seen as the spatial separation of the catalyst bed into its upper section (which receives most of the heat during regeneration and reduction of the catalyst bed and thus supplies most of the heat to the dehydrogenation reaction) located in the first reactor and its lower section (which receives and provides less heat to the dehydrogenation reaction accordingly) located in the second reactor. In case the olefin stream S2 is fed into the first reactor the additional heat provided by the exothermic hydrogenation reaction of the olefin is preferably distributed to both reactors. In this way the temperature drop in both reactors is decreased. The degree of the temperature drop depends on the amount of olefin stream S2 supplied to the first reactor. In case the olefin stream S2 is supplied to the second reactor, the temperature drop in the second reactor alone will be decreased and as the result the temperature in the second reactor will have the same or a higher temperature than the first reactor.

In an embodiment of said multiple reactor arrangement for conducting the present process the at least one first alkane stream S1 is fed solely into the first reactor and is only mixed with the at least one second olefin stream S2 after leaving the first reactor and before entering the second reactor. Thereby it is preferred if the at least one first alkane stream S1 is fed into a first reactor as front feed and the at least one second olefin stream S2 is fed into the gas stream leaving the first reactor at at least one location between the first reactor and the second reactor.

It is also possible that the at least one olefin stream S2 is fed to the reactor system at at least one location alongside of the first reactor and/or the second reactor.

According to the invention the at least one alkane stream leaving the first reactor (intermediate effluent stream) having a reduced temperature due to the heat consumption during the endothermic dehydrogenation is re-heated by passing at least one heater and the heated intermediate effluent stream leaving the heater is fed into the second reactor.

It is also according to the invention that the at least one second olefin stream is heated before entering the second reactor. Thus, the at least one olefin stream for example ethene stream may be introduced into the heater and may be thereby be mixed with the intermediate effluent stream in the heater or before entering the heater. In the latter case a mixed stream of intermediate effluent stream and olefin is fed into the heater.

Both, the heating of the alkane and the olefin stream may be carried out in a heater arranged between the first reactor and the second reactor.

The mixed stream of intermediate effluent stream and olefin is - after leaving the heater-subsequently fed into the second reactor wherein further dehydrogenation reaction of the non-saturated alkane such propane to the corresponding olefin such as propene takes place. The hydrogen released during the dehydrogenation reaction reacts with the olefin such as ethene in an exothermic hydrogenation reaction producing the corresponding saturated alkane such as ethane. The heat released during the exothermic reaction is used in turn for the endothermic dehydrogenation reaction.

In general the reactor effluent - either from the single reactor or the multiple reactor system-comprises a reaction mixture of newly produced olefin (dehydrogenated alkane) from the first alkane stream and hydrogenated olefin (saturated alkane) from the second olefin stream as well as small amounts of hydrogen. Other by-products may also be present.

In the present case of propane dehydrogenation according to the invention propane is converted to propene and the co-fed ethene is converted to ethane; in case of iso-butane dehydrogenation iso-butane is converted to iso-butene and the co-fed ethene is converted to ethane, or in case of ethylbenzene dehydrogenation ethylbenzene is converted to styrene and the co-fed ethene converted to ethane.

Furthermore, the present process does not depend on a specific reactor cycle. Thus, the present process can be used in all dehydrogenation cycle lengths, regeneration cycle lengths and/or reduction phase length.

The present invention is further explained in more detail based on the following examples in conjunction with the Figures. It shows:
- Fig. 1a: a schematic view of a first process variant in a single reactor according to the invention;
- Fig. 1b: a schematic illustration of a time line according to the first process variant;
- Fig. 2a: a schematic view of a second process variant in a single reactor according to the invention;
- Fig. 2b: a schematic view of a second process variant in a multiple reactor system;
- Fig. 3: a diagram showing the temperature profile of the catalytic bed for an embodiment of the present process;
- Fig. 4a: a first schematic view of a first process variant adapted for a Catofin Process;
- Fig. 4b: a second schematic view of a first process variant adapted for a Catofin Process; and
- Fig. 5: a schematic view of a second process variant adapted for an Oleflex process.

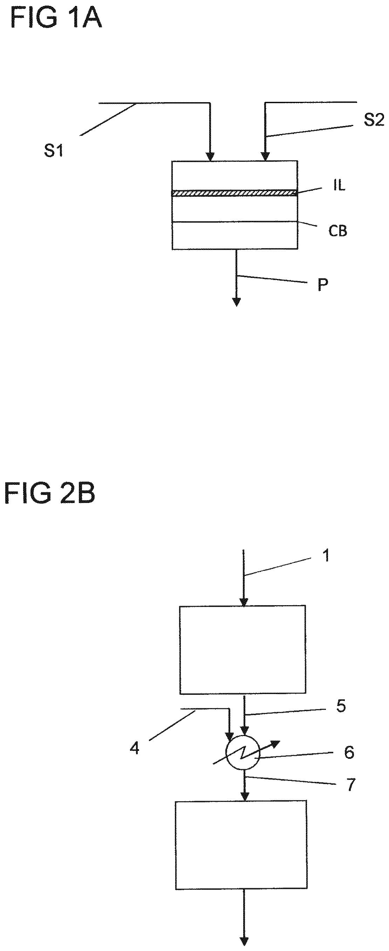

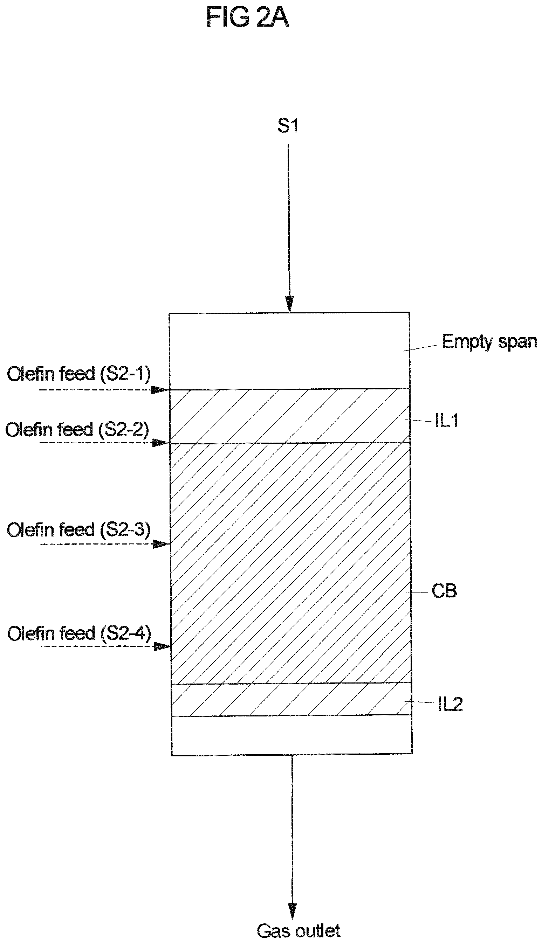

Figure 1a shows schematically a first variant of the present process conducting in a single reactor R comprising a first inert layer IL1, a catalyst bed CB made of a mixture of dehydrogenation catalyst and inert material, and a second inert layer IL2 (shown in Fig. 2A). The alkane stream S1 and the olefin stream S2 are fed into the single reactor R. When entering the catalyst bed CB the alkane (such as propane) is dehydrogenated to the corresponding alkene (such as propene) and the olefin (such as ethene) is hydrogenated to the corresponding alkane (such as ethane). The reactor effluent stream P leaving the reactor R comprises dehydrogenated alkane and hydrogenated olefin and small amounts of non-reacted starting material as well as other by-products.

Alkane stream S1 and olefin stream S2 are fed to the reactor at different times. Typically the dehydrogenation process is started by feeding alkane (such as propane) stream S1 at a time t_{S1(0)}. Immediately when feeding the alkane to the reactor dehydrogenation (dehy) begins. Olefin stream S2 (such as ethene) is fed to the reactor at t_{S2(0)} which is typically delayed after the beginning of dehydrogenation.

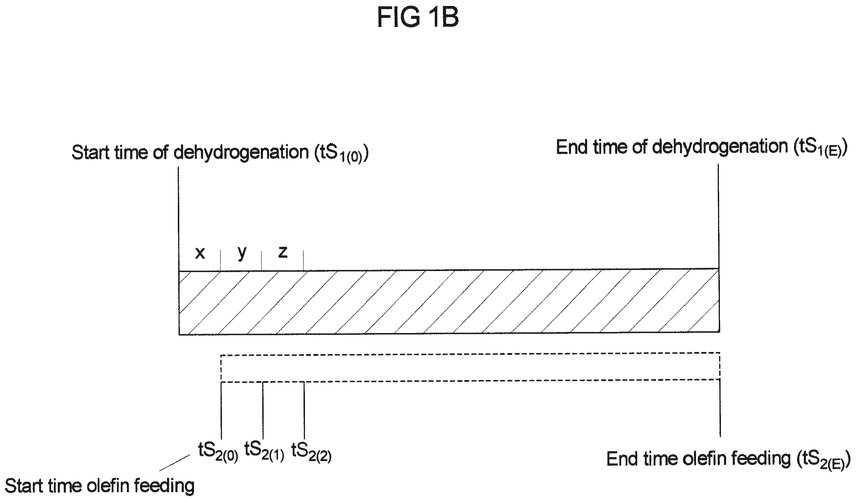

The ratio of dehydrogenation time and olefin feeding time is schematically shown in Fig. 1b. It is assumed that total continuous alkane feeding time tS₁ (or dehydrogenation), which begins at tS₁₍₀₎ = 0 sec and ends at tS_{1(E)}, is 10 min.

After 1 min (x min) of dehydrogenation at t_{S1(1}) olefin feeding S2 is started (tS₁₍₁₎ = tS₂₍₀₎ = t S1(0) + 1 min). The olefin feeding S2 may then be continued until the dehydrogenation reaction is ended, e.g. for instance until tS_{1(E)} is reached.

In this case, when the time delay x of starting the olefin feed S1 is 1 min, the total olefin feeding time tS₂ is 9 min. Thus, the ratio of total olefin feeding time tS₂ and total alkane feeding time tS₁ would be 0.9.

It is also possible to interrupt the olefin feeding S2 at any time, for instance at tS₂₍₁₎ = tS₂₍₀₎ + 1 min (y min)) for 1 min (z min) and restart the olefin feeding at tS₂₍₂₎ = tS₂₍₀₎ + 2 min (y + z min). Such an interruption of olefin feeding would have the advantage of reducing the overall coke formation.

It is furthermore possible to stop the olefin feeding before the end of dehydrogenation at tS_{1(E)}. For instance in the last minute of the dehydrogenation reaction there is no olefin feed necessary, since the additional heat created by the olefin hydrogenation would be lost.

Figure 2a shows schematically the second variant of the present process. Here the alkane feeding S1 and olefin feeding S2 are carried at spatially separated locations. In the embodiment of Fig. 2a the alkane feed (such as propane feed) S1 is fed into the reactor R as front feed. The olefin feed (such as ethen feed) S2 can be fed at one or multiple locations alongside of the reactor R. For example, alkane S2 can be fed to the top of the first inert layer IL1 as S2-1, to the top of the catalyst bed CB as S2-2, to the middle of catalyst bed CB as S2-3 and/or at some point to the second half of the catalyst bed CB as S2-4. Since the olefin can be fed at multiple points it may provide flexibility in reaching isothermality at particular sections in a catalyst bed.

Figure 2b shows the second process variant conducted in a multiple reactor system. This reactor system comprises two reactors R1, R2, which are connected in series. In each reactor a catalyst bed comprising at least one inorganic catalytic material and at least one first inert material is provided.

The alkane stream S1 is fed into the first reactor R1 and enters the catalyst bed CB where dehydrogenation of the alkane proceeds. The stream leaving the first reactor (intermediate effluent stream IS) is then passed through a heater H, which is arranged between first reactor R1 and second reactor R2.

Furthermore, the olefin stream S2 is also fed into the heater H. Intermediate effluent stream IS and olefin feed S2 are thus mixed in the heater H.

The mixed stream of intermediate effluent stream IS and olefin feed S2 is - after leaving the heater- subsequently fed into the second reactor R2 wherein further dehydrogenation reaction of the alkane to the corresponding olefin place. The hydrogen released during the dehydrogenation reaction reacts with the olefin of the olefin feed S2 in an exothermic hydrogenation reaction producing the corresponding saturated alkane. The heat released during the exothermic reaction is used in turn for the endothermic dehydrogenation reaction.

The co-feeding of an olefin improves the overall conversion of the alkane to be dehydrogenated.

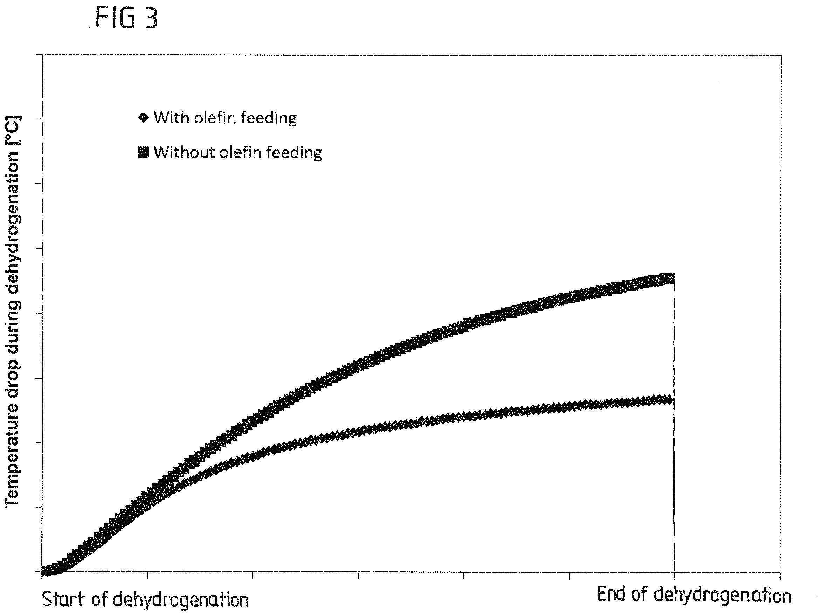

The diagram of Figure 3 shows the influence of the co-fed olefin (here ethene) onto the temperature gradient within the catalyst bed.

In a conventional process (upper curve without co-feeding ethylene) there is a strong temperature drop from the upper section to the lower section of the catalyst bed. This is due to the fact that the upper section of the catalyst bed gets most of the heat during regeneration and reduction steps and supplies most of the heat to the reaction during the dehydrogenation reaction. On the other hand the heat consumed and supplied by the lower sections of the bed is quite low compared to the upper sections.

When co-feeding an alkane such as ethene the temperature drop over the catalyst bed is less predominant (lower curve of the diagram in Figure 3). In fact the temperature drop is drastically reduced such that the temperature difference between upper and lower section in the catalyst bed can almost be neglected.

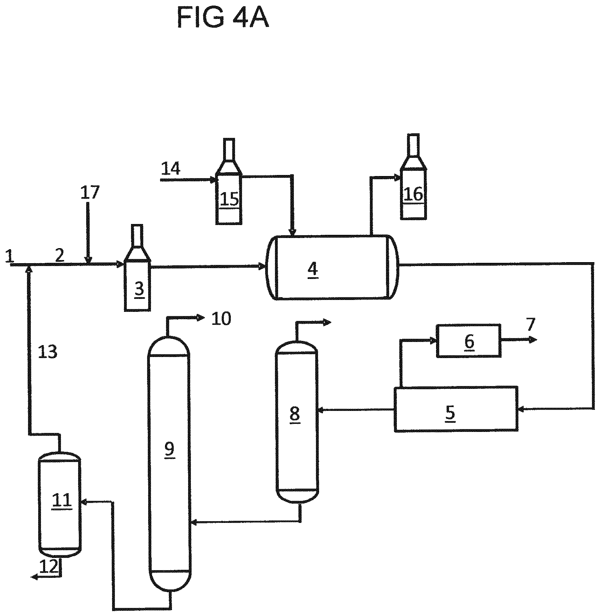

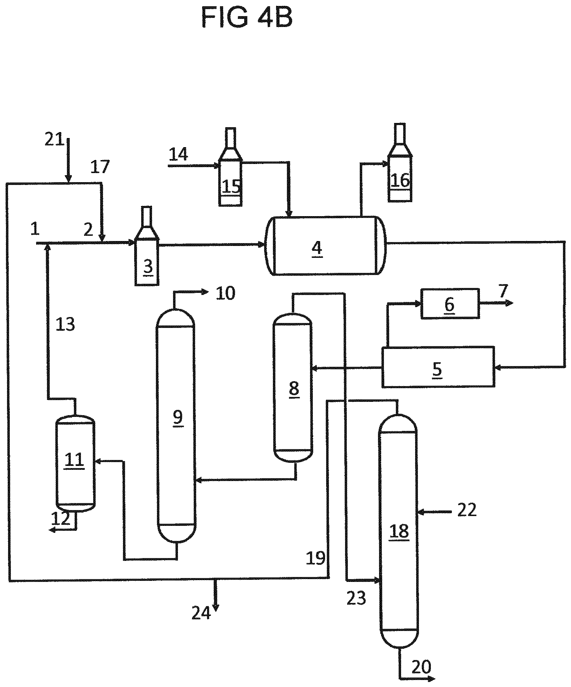

Figures 4a and 4b show two embodiments of the first process variant adapted for a Catofin Process for propane dehydrogenation, respectively. Fresh propane feed (1) is mixed with the recycle propane feed (13) to form a combined feed (2) and fed to a fired heater (3). The heated feed is then reacts in reactor (4) in the presence of a catalyst. The product gas mixture along with unreacted propane goes to the cold section where light gases are separated. The hydrogen (7) from the light gases is then separated from the remaining light gases using a pressure swing adsorption unit (6). The liquid from the cold box is then sent to a de-ethaniser (8) where components lighter than propylene are removed at the top. Propylene and the other heavier components are sent to a C3 splitter tower (9) where propylene (10) is obtained at the top and propane and the remaining components are sent to a deoiler tower (11) where components heavier than propane (12) are removed at the bottom and propane is recycled back. To regenerate the catalyst, regeneration air (14) is sent through air heater to the reactors. After regeneration, the air is sent to a waste heat boiler.

According to the embodiment of Fig. 4a ethylene feed (17) is also supplied along with fresh and recycle propane. In the embodiment of Figure 4b, the gas mixture (23) from the top of de-ethaniser (8) is sent to a further separation section (18) where ethylene (19) is purified and supplied back to the propane feed. Optionally, the separation section (18) can also get feed (22) from different plants, for example from a cracker or from a refinery. Also in the present process a part of ethylene from the separation section (18) can be removed as a product. Optionally, an additional ethylene supply (21) can also be used with the ethylene feed from separation section (18).

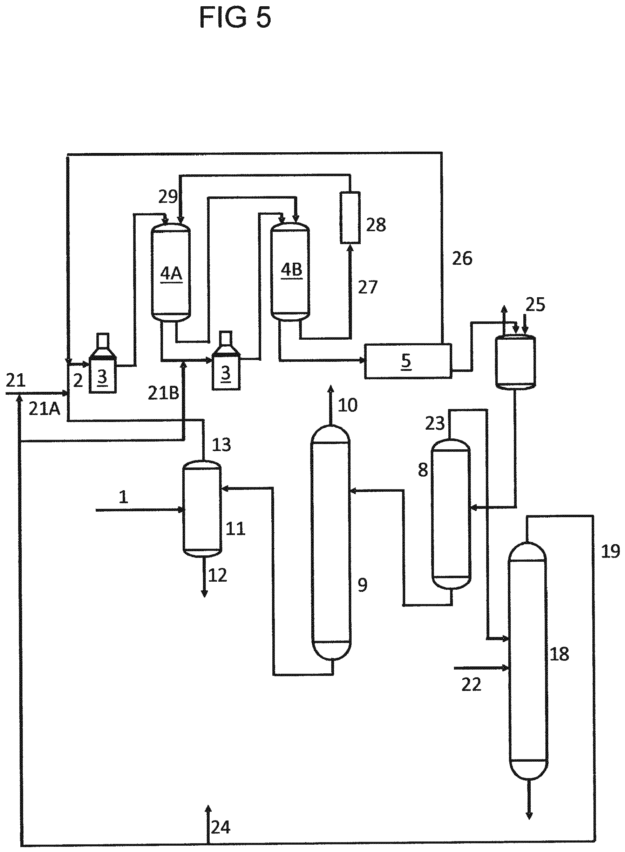

Fig. 5 is schematic view of a second process variant adapted for an Oleflex process. In the Oleflex process, multiple reactors are connected in series. Though 2 reactors (4A and 4B) are shown in this application, the number of reactors could be more. In Oleflex process, the fresh propane (1) and recycle propane which contains heavier components are fed to a depropaniser (11) in which propane rich gas stream (13) is obtained at the top of depropaniser (11). The propane rich gas stream is mixed together with a recycle hydrogen stream (26) and heated in a fired heater (3A). The heated gas stream is fed to the first moving bed reactor (4A) where the gas stream reacts in the presence of catalyst (29). The gas stream which exits the reactor (31) is heated again in a heater (3B) before being sent to the next reactor (4B). The gas stream from the final reactor is sent to a separation section (5) where hydrogen (26) is removed from the rest and recycled back to the reactor (4A). The liquid from the separation section (5) is then fed to a selective hydrogenation reactor section (33). The liquid stream from 5 is fed to a deethaniser where components lighter than propylene are removed. Propylene along with heavier components is then fed to a C3 splitter (9) where propylene product (10) is obtained from the top. The bottom stream of C3 splitter is then fed to depropaniser (11) where the components heavier then propane (12) is removed at the bottom. The used catalyst (27) from the final reactor is regenerated in a regeneration section (28) and recycled back (29) to the first reactor.

According to the embodiment of the second process variant shown in Fig. 5 ethylene feeds (21A and 21B) are fed along with propane/hydrogen gas mixture and with the intermediate product stream (31). The ethylene feed can be obtained from an ethylene purification section (8) included in the process or from a different source (21). Also optionally ethylene feed (21A and 21B) can be obtained by the combination of recycle stream 19 and fresh source 21. The fresh source can have impurities of ethane. The ethylene purification section (18) can have additional feed (22) from a different process or source which can be a mixture of ethylene and ethane.

## Claims

1. Endothermic catalytic propane dehydrogenation process conducted in gas phase in at least one reactor system comprising at least two reactors (R1, R2) that are connected in series and comprise at least one catalyst bed (CB1, CB2) comprising at least one inorganic catalytic material and at least one first inert material, respectively, comprising the steps of:
- feeding at least one first stream S1 comprising propane into the first reactor (R1) as front feed, and
- feeding at least one second stream S2 comprising ethylene into the gas stream leaving the first reactor (R1) at at least one location between the first reactor (R1) and the second reactor (R2),
wherein the gas stream leaving the first reactor (R1) and the at least one second stream S2 comprising ethylene are mixed in / or before entering at least one heater (H) arranged between first reactor (R1) and second reactor (R2) and heated in the heater (H) before entering the second reactor (R2).

2. Process according to one of the preceding claims, **characterized in that** at least one layer of a second inert material (IL) is arranged upstream and/or downstream of at least one of the catalyst bed (CB1, CB2) in at least one of the reactors (R1, R2).

3. Process according to claim 2, **characterized in that** the second olefin stream S2 is fed to the at least one layer of a second inert material (IL) arranged upstream and/or downstream of at least one of the catalyst beds (CB1, CB2) in at least one of the reactors (R2).

4. Process according to one of the preceding claims, **characterized in that** the temperature of the propane feed S1 and of the ethylene feed S2 are between 400 and 650 °C, preferably 500 and 650 °C, most preferably 550 and 650 °C, respectively, and that reaction temperatures in the catalyst bed are between 500 and 1000°C, preferably between 500 and 800 °C, most preferably between 500 and 700 °C.

5. Process according to one of the preceding claims, **characterized in that** the at least one inorganic catalyst material of the catalyst bed (CB, CB1, CB2) is selected from a group chromium oxide, platin, iron, vanadium or a mixture thereof and the at least one first inert material of the catalyst bed is selected magnesium oxide, aluminium oxide, aluminium nitride, titanium oxide, zirconium dioxide, niobium oxide and aluminium silicate.

## Patentansprüche

1. Endothermes katalytisches Dehydrierungsverfahren von Propan, das in der Gasphase in mindestens einem Reaktorsystem durchgeführt wird, das mindestens zwei Reaktoren (R1, R2) umfasst, die in Reihe geschaltet sind und mindestens ein Katalysatorbett (CB1, CB2) umfassen, das jeweils mindestens ein anorganisches katalytisches Material und mindestens ein erstes inertes Material umfasst, umfassend die Schritte von:
- Zuführen von mindestens einem ersten Strom S1, der Propan umfasst, in den ersten Reaktor (R1) als Frontzufuhr, und
- Zuführen von mindestens einem zweiten Strom S2, der Ethylen umfasst, in den Gasstrom, der den ersten Reaktor (R1) an mindestens einer Stelle zwischen dem ersten Reaktor (R1) und dem zweiten Reaktor (R2) verlässt,
wobei der Gasstrom, der den ersten Reaktor (R1) verlässt, und der mindestens eine zweite Strom S2, der Ethylen umfasst, in / oder vor dem Eintritt in mindestens ein Heizgerät (H) gemischt werden, das zwischen dem ersten Reaktor (R1) und dem zweiten Reaktor (R2) angeordnet ist, und in dem Heizgerät (H) vor dem Eintritt in den zweiten Reaktor (R2) erhitzt wird.

2. Verfahren nach einem der vorstehenden Ansprüche, **dadurch gekennzeichnet, dass** mindestens eine Schicht eines zweiten inerten Materials (IL) stromaufwärts und/oder stromabwärts von mindestens einem der Katalysatorbetten (CB1, CB2) in mindestens einem der Reaktoren (R1, R2) angeordnet ist.

3. Verfahren nach Anspruch 2, **dadurch gekennzeichnet, dass** der zweite Olefinstrom S2 in die mindestens eine Schicht eines zweiten inerten Materials (IL) zugeführt wird, das stromaufwärts und/oder stromabwärts von mindestens einem der Katalysatorbetten (CB1, CB2) in mindestens einem der Reaktoren (R2) angeordnet ist.

4. Verfahren nach einem der vorstehenden Ansprüche, **dadurch gekennzeichnet, dass** die Temperatur der Propanzufuhr S1 und der Ethylenzufuhr S2 jeweils zwischen 400 und 650°C, vorzugsweise 500 und 650°C, am meisten bevorzugt 550 und 650°C liegt und dass die Reaktionstemperaturen im Katalysatorbett zwischen 500 und 1000°C, vorzugsweise zwischen 500 und 800°C, am meisten bevorzugt zwischen 500 und 700°C liegen.

5. Verfahren nach einem der vorstehenden Ansprüche, **dadurch gekennzeichnet, dass** das mindestens eine anorganische Katalysatormaterial des Katalysatorbettes (CB, CB1, CB2) ausgewählt ist aus einer Gruppe Chromoxid, Platin, Eisen, Vanadium oder einer Mischung davon und das mindestens eine erste inerte Material des Katalysatorbettes ausgewählt ist Magnesiumoxid, Aluminiumoxid, Aluminiumnitrid, Titanoxid, Zirkoniumdioxid, Nioboxid und Aluminiumsilikat.

## Revendications

1. Procédé de déshydrogénation catalytique endothermique du propane effectué en phase gazeuse dans au moins un système de réacteurs comprenant au moins deux réacteurs (R1, R2) qui sont reliés en série et comprennent au moins un lit catalytique (CB1, CB2) comprenant au moins un matériau catalytique inorganique et au moins un premier matériau inerte, comprenant respectivement les étapes consistant :
- à fournir au moins un premier courant S1 comprenant du propane dans le premier réacteur (R1) en tant que charge d'alimentation frontale, et
- à fournir au moins un deuxième courant S2 comprenant de l'éthylène dans le courant de gaz sortant du premier réacteur (R1) à au moins un emplacement entre le premier réacteur (R1) et le deuxième réacteur (R2),
dans lequel le courant de gaz sortant du premier réacteur (R1) et l'au moins un deuxième courant S2 comprenant de l'éthylène sont mélangés pendant / ou avant l'entrée dans au moins un appareil de chauffage (H) agencé entre le premier réacteur (R1) et le deuxième réacteur (R2) et chauffés dans le l'appareil de chauffage (H) avant d'entrer dans le deuxième réacteur (R2).

2. Procédé selon la revendication 1, **caractérisé en ce qu'**au moins une couche d'un deuxième matériau inerte (IL) est agencée en amont et/ou en aval d'au moins l'un des lits catalytiques (CB1, CB2) dans au moins l'un des réacteurs (R1, R2).

3. Procédé selon la revendication 2, **caractérisé en ce que** le deuxième courant d'oléfine S2 est fourni à l'au moins une couche d'un deuxième matériau inerte (IL) agencée en amont et/ou en aval d'au moins l'un des lits catalytiques (CB1, CB2) dans au moins l'un des réacteurs (R2).

4. Procédé selon l'une des revendications précédentes, **caractérisé en ce que** les températures de la charge d'alimentation en propane S1 et de la charge d'alimentation en éthylène S2 sont comprises entre 400 et 650°C, de préférence entre 500 et 650 °C, idéalement entre 550 et 650 °C, respectivement, et **en ce que** les températures de réaction dans le lit catalytique sont comprises entre 500 et 1000°C, de préférence entre 500 et 800 °C, idéalement entre 500 et 700 °C.

5. Procédé selon l'une des revendications précédentes, **caractérisé en ce que** l'au moins un matériau catalytique inorganique du lit catalytique (CB, CB1, CB2) est choisi dans le groupe constitué par l'oxyde de chrome, le platine, le fer, le vanadium ou un mélange de ceux-ci et l'au moins un premier matériau inerte du lit catalytique est choisi parmi l'oxyde de magnésium, l'oxyde d'aluminium, le nitrure d'aluminium, l'oxyde de titane, le dioxyde de zirconium, l'oxyde de niobium et le silicate d'aluminium.
